# FASCICULE DE BREVET EUROPEEN

(11) **EP 3 366 297 B1**
(45) Date de publication et mention de la délivrance du brevet: **18.12.2019**
(21) Numéro de dépôt: 18156486.5
(22) Date de dépôt: 13.02.2018
(51) Int. Cl.: A61K 33/18, A61K 31/79, A01N 25/02, A01N 59/12, A01N 25/04, A01N 25/10, A61P 31/02

(54) **COMPOSITION DESINFECTANTE ET FILMOGENE DESTINEE A ETRE APPLIQUEE APRES LA TRAITE SUR UN TRAYON CONTENANT UN IODOPHORE**
DESINFIZIERENDE UND FILMBILDENDE ZUSAMMENSETZUNG MIT EINEM IODOPHOR ZUR AUFBRINGUNG NACH DEM MELKEN AUF EINE EUTERZITZE
DISINFECTANT AND FILM-FORMING COMPOSITION INTENDED FOR BEING APPLIED AFTER MILKING ON A TEAT CONTAINING IODOPHOR

(30) Priorité: 23.02.2017 FR 1751432
(43) Date de publication de la demande: 29.08.2018
(73) Titulaire: Hydrachim, 35370 Le Pertre (FR)
(72) Inventeur: FRETIN, Benoît, 35370 LE PERTRE (FR); GABLIN, Sandra, 53320 SAINT CYR LE GRAVELAIS (FR); LEBRETON, Rodrigue, 53320 SAINT CYR LE GRAVELAIS (FR)
(74) Mandataire: Ermeneux, Bertrand

(56) Documents cités:
- US-A- 5 641 498
- US-A- 5 720 984

## Description

### 1. Domaine de l'invention

Le domaine de l'invention est celui de l'hygiène de la traite.

Plus précisément, l'invention concerne une composition désinfectante et filmogène destinée à être appliquée après la traite sur les trayons d'un bovin, d'un ovin ou d'un caprin.

### 2. Etat de la technique

Lors de la traite, une attention particulière doit être apportée à la désinfection des trayons et des soins cutanés doivent être administrés. Ceci permet de garantir la qualité du lait et de préserver la santé de l'animal.

Ces traitements consistent essentiellement à appliquer sur les trayons une solution liquide désinfectante, adoucissante et émolliente, par trempage ou par pulvérisation. L'application de cette solution vise à éliminer les germes, et notamment les germes *d'Escherichia Coli,* de *Streptococcus uberis* ou de *Staphylococcus aureus* présents à la surface de la peau des trayons. Elle permet également de soulager les irritations provoquées par la machine de traite et de soigner les éventuelles crevasses et gerçures sur la peau du trayon, qui peuvent entraîner des mammites en cas d'infection bactérienne.

Dans les élevages bovins, ovins ou caprins, il est connu d'appliquer, un produit désinfectant et filmogène sur les trayons après la traite, destiné à protéger, jusqu'à la prochaine traite, la peau et le sphincter des trayons des contaminations bactériennes pouvant survenir, par exemple, au contact d'une litière souillée. Ce produit qui forme un film après séchage, constitue une barrière physique contre les microorganismes, qui les empêche notamment de coloniser le canal des trayons et les sinus galactophores.

On connait des compositions désinfectantes et filmogènes s'appliquant après la traite, dont la substance active bactéricide est un iodophore et/ou de la chlorexhidine.

Le document EP-B1-0 915 658 propose par exemple d'utiliser de la polyvinylpyrrolidone-iode. Celle-ci est mélangée en solution aqueuse avec un polymère d'alcool vinylique, qui agit comme agent filmogène. Pour obtenir un film d'épaisseur homogène, de l'acide carboxylique d'éther, tel que de l'acide caprylique, est incorporé à la solution.

Un inconvénient des acides carboxyliques d'éther est qu'ils oxydent la polyvinylpyrrolidone-iode ce qui réduit son efficacité bactéricide dans la solution.

Un autre inconvénient des formulations proposées dans le document EP-B1-0 915 658 est que le film qui se forme après application et séchage de la solution est gras, ce qui le rend difficile à enlever des trayons.

Le document US-A-5,720,984 décrit une composition à appliquer sur les trayons d'un animal comprenant 1,5% d'un iodophore, 10% de glycérine, 10% d'acide laureth (11-16) carboxylique, de l'acide citrique en tant que tampon pH et de l'eau,
On connait également du document US-A-5,641,498 une composition désinfectante et filmogène s'appliquant après la traite dont la substance active bactéricide est de la chlorexhidine et l'agent filmogène est un alcool polyvinylique. Cette formulation contient un tensioactif non ionique de type éthoxylate d'acide gras destiné à permettre une meilleure répartition de la formulation sur la surface du trayon pour obtenir un film d'épaisseur homogène.

Un inconvénient de cette formulation est que le film obtenu est collant et se retire très difficilement.

### 3. Objectifs de l'invention

L'invention a donc notamment pour objectif de pallier les inconvénients de l'état de la technique cités ci-dessus.

Plus précisément l'invention a pour objectif de fournir une composition désinfectante et filmogène destinée à être appliquée après la traite dont la substance active bactéricide est un iodophore, qui soit efficace en quelques minutes contre les micro-organismes pathogènes, tels que les bactéries et les levures, et notamment les germes *d'Escherichia Coli,* de *Streptococcus uberis* ou de *Staphylococcus aureus* et l'espèce de levure *Candida albicans,* tout en contenant une quantité d'iode limitée. Le pouvoir désinfectant des compositions est dans le cadre de l'invention évalué conformément aux méthodes d'essais mentionnées dans les normes EN 1656 et EN 1657 en conditions de désinfection des trayons.

Un objectif est notamment, dans un mode de réalisation particulier de l'invention, d'obtenir une composition désinfectante et filmogène contenant moins de 1% d'iode qui soit efficace contre des microorganismes pathogènes.

Un objectif de l'invention est également de fournir une telle composition qui forme un film régulièrement réparti et d'épaisseur homogène sur le trayon.

L'invention a également pour objectif de proposer une composition désinfectante et filmogène qui puisse être retirée facilement du trayon par un simple lavage et un essuyage humide avant la traite suivante, sans risque d'abrasion ou d'irritation du trayon.

Encore un objectif de l'invention est également de proposer une composition désinfectante et filmogène destinée à être appliquée après la traite qui soit d'un coût de revient réduit.

L'invention a par ailleurs pour objectif de fournir une composition désinfectante et filmogène qui soit efficace contre les microorganismes sur l'intervalle de temps entre deux traites, soit entre 8 et 12 heures.

Un autre objectif de l'invention est de fournir une composition désinfectante et filmogène destinée à être appliquée après la traite qui ne provoque pas de réactions d'intolérance et d'irritations.

Encore un objectif de l'invention est de fournir une telle composition qui soit stable dans le temps et à des températures comprises entre 5°C et 25°C.

L'invention a également pour objectif de fournir une composition désinfectante et filmogène après-traite qui soit non toxique et non allergène pour l'animal ou pour l'éleveur.

### 4. Exposé de l'invention

La Demanderesse a découvert, de façon surprenante et inattendue, en cherchant à formuler une composition désinfectante et filmogène destinée à être appliquée après une traite sur le trayon d'un bovin, d'un ovin ou d'un caprin comprenant en milieu aqueux 3,5 à 5% en masse de l'acétate de vinyle polymérisé avec l'alcool vinylique, comme composant filmogène, et 0,75 à 2,9 % en masse d'un complexe de polyvinylpyrrolidone et d'iode comme substance active désinfectante, permettant de répondre à ces objectifs, qu'une composition comprenant en outre 0,2 à 0,6% en masse de polymère d'oxyde de propylène et d'oxyde d'éthylène et 0,5 à 1% en masse de lauryl éther sulfate de sodium permettait d'y parvenir.

L'invention propose ainsi, de façon inédite, d'utiliser du polymère d'oxyde de propylène et d'oxyde d'éthylène, qui est un tensio-actif non-ionique qui ne dégrade pas l'iode, pour faciliter la formation d'un film homogène à la surface du trayon, après séchage.

Les inventeurs ont par ailleurs constaté que l'action polysynergique du polymère d'oxyde de propylène et d'oxyde d'éthylène et du tensio-actif anionique lauryl éther sulfate de sodium avec l'acétate de vinyle polymérisé avec l'alcool vinylique permet d'obtenir une solution qui ne goutte pas et qui se répartit de façon homogène à la surface du trayon.

Dans ces conditions, l'acétate de vinyle polymérisé avec l'alcool vinylique, aussi connu sous l'acronyme PVA, forme, après séchage, un film souple et fin, qui autorise la respiration de la peau, tout en formant une barrière de protection contre les salissures.

Ce film polymérique non réticulé est suffisamment résistant pour permettre l'action prolongée du complexe de polyvinylpyrrolidone et d'iode entre deux traites, et suffisamment soluble dans l'eau et non collant pour être éliminé par un simple lavage lors de la préparation de la traite suivante.

Il est par ailleurs résistant aux frottements et aux conditions extérieures, telles que le froid, la pluie ou le vent.

Enfin il sèche rapidement.

Avantageusement, la proportion massique du polymère d'oxyde de propylène et d'oxyde d'éthylène dans ladite composition est comprise entre 0,25 et 0,35 %.

De préférence, ledit acétate de vinyle polymérisé avec l'alcool vinylique est hydrolysé à au moins 98%.

Dans un mode de réalisation particulier de l'invention, une composition désinfectante et filmogène telle que décrite ci-dessus comprend 0,5 à 2% de PEG-7 glyceryl cocoate, qui assure notamment une fonction d'activateur lipidique de l'épiderme et d'émollient.

De façon avantageuse, une composition désinfectante et filmogène telle que décrite ci-dessus comprend 3 à 10% en masse de glycérine.

Préférentiellement, une telle composition désinfectante et filmogène comprend 0,05 à 1% en masse d'un tampon de pH.

Le tampon de pH peut par exemple être du citrate trisodique dihydraté, du lactate de sodium, du phosphate tripotassique anhydre, ou du N,Nbis(carboxyméthyl)-DL-alanine, triple sel de sodium. Il permet de garantir une bonne stabilité de la composition dans le temps et de maintenir une concentration active en iode suffisante.

Selon un aspect particulier de l'invention, une composition désinfectante et filmogène telle que décrite ci-dessus comprend 0,1 à 0,5% en masse d'un agent régulateur de pH ou d'un mélange de plusieurs agents régulateurs de pH en mélange, destinés à ajuster le pH de ladite composition entre 4,5 et 5,5.

Cet agent régulateur de pH ou ce mélange d'agents régulateurs de pH permet avantageusement d'ajuster la valeur du pH de la composition à une valeur proche du pKa de l'iode.

Le régulateur de pH peut par exemple être de l'hydroxyde de sodium, de l'iminodisuccinate de sodium et/ou de l'acide citrique monohydrate.

Dans un mode de réalisation particulier de l'invention, ledit mélange d'agents régulateur de pH comprend de l'hydroxyde de sodium et de l'acide citrique monohydrate.

Dans un mode de réalisation particulier de l'invention, une composition désinfectante et filmogène telle que décrite ci-dessus comprend de la gomme de xanthane, destinée à épaissir ladite composition.

Il peut également être envisagé de mettre en œuvre de la carboxyméthylcellulose et/ou de la pectine pour épaissir la composition désinfectante et filmogène, dans un autre mode de réalisation de l'invention.

Cet agent épaississant permet d'ajuster la viscosité de la composition de façon à permettre une application aisée de la composition par trempage, sans dépôt excessif, mais également sans perdre de produit, par gouttage, lorsque la composition sèche et que le film se forme.

Dans un mode de réalisation particulier de l'invention, une composition désinfectante et filmogène telle que décrite ci-dessus comprend 0,01 à 0,5% en masse d'allantoïne.

### 5. Exemples de composition selon l'invention

On a détaillé dans le tableau ci-dessous un exemple préféré de composition désinfectante et filmogène selon l'invention, qui forme un film homogène, particulièrement facile à retirer des trayons.

| **Composé** | **% massique** |
|---|---|
| Complexe de polyvinylpyrrolidone et d'iode | 1,5 % |
| polymère d'oxyde de propylène et d'oxyde d'éthylène | 0,3 % |
| PEG-7 glyceryl cocoate | 2 % |
| Acide citrique monohydrate | 0,09 % |
| Citrate trisodique dihydraté | 0,1% |
| Hydroxyde de sodium | 0,5394 % |
| Acétate de vinyle polymérisé avec l'alcool vinylique, complètement hydrolysé | 4,396 % |
| lauryl éther sulfate de sodium | 0,9396% |
| Glycérine | 8 % |
| Allantoïne | 0,5 % |
| Méthanol | 0,13188% |
| Gomme de xanthane | 0,4 % |
| Colorants | 0,04 % |
| Eau déminéralisée | q.s.p. 100% |

Des tests suivant les normes EN 1656 et 1657 ont montré que cette composition est efficace en 5 minutes à 30°C dans des conditions de désinfection des trayons contre les souches *d'Escherichia Coli,* de *Streptococcus uberis* ou de *Staphylococcus aureus* et qu'elle inactive *Candida albicans.*

Un autre exemple de composition désinfectante et filmogène selon l'invention plus concentrée en complexe de polyvinylpyrrolidone et d'iode est donné dans le tableau ci-après.

| **Composé** | **% massique** |
|---|---|
| Complexe de polyvinylpyrrolidone et d'iode | 2,9 % |
| polymère d'oxyde de propylène et d'oxyde d'éthylène | 0,3 % |
| PEG-7 glyceryl cocoate | 2 % |
| Acide citrique monohydrate | 0,09 % |
| Citrate trisodique dihydraté | 0,1 % |
| Hydroxyde de sodium | 0,93 % |
| Acétate de vinyle polymérisé avec l'alcool vinylique, complètement hydrolysé | 4,396 % |
| lauryl éther sulfate de sodium | 0,9396% |
| Glycérine | 8 % |
| Allantoïne | 0,5 % |
| Méthanol | 0,13188% |
| Gomme de xanthane | 0,4 % |
| Colorants | 0,04 % |
| Eau déminéralisée | q.s.p. 100% |

Des tests suivant les normes EN 1656 et 1657 ont montré que cette composition est efficace en 2 minutes à 30°C dans des conditions de désinfection des trayons contre les souches d'*Escherichia Coli,* de *Streptococcus uberis* ou de *Staphylococcus aureus* et qu'elle inactive *Candida albicans.*

Un autre exemple de composition désinfectante en 5 minutes et filmogène selon l'invention est donné dans le tableau ci-dessous :

| **Composé** | **% massique** |
|---|---|
| Complexe de polyvinylpyrrolidone et d'iode | 1,50 % |
| polymère d'oxyde de propylène et d'oxyde d'éthylène | 0,20 % |
| PEG-7 glyceryl cocoate | 2,00 % |
| Acide citrique monohydrate | 0,09 % |
| Citrate trisodique dihydraté | 0,10 % |
| Hydroxyde de sodium | 0,45 % |
| Acétate de vinyle polymérisé avec l'alcool vinylique, complètement hydrolysé | 3,50 % |
| lauryl éther sulfate de sodium | 1,00 % |
| Glycérine | 3,00 % |
| Allantoïne | 0,50 % |
| Méthanol | 0,10502% |
| Gomme de xanthane | 0,40 % |
| Colorants | 0,04 % |
| Eau déminéralisée | q.s.p. 100% |

Cette formulation est moins concentrée en complexe de polyvinylpyrrolidone et d'iode que la précédente et plus faiblement filmogène, et elle assure une protection des trayons pendant au moins 1 heure.

Les inventeurs ont constaté qu'il était nécessaire pour obtenir une protection optimale, permettant de récupérer un film à la surface de la peau des trayons, que les proportions massiques minimales suivantes en polymère d'oxyde de propylène et d'oxyde d'éthylène et en lauryl éther sulfate de sodium soient respectées dans la formulation des compositions selon l'invention :
- pour 4% en masse d'acétate de vinyle polymérisé avec l'alcool vinylique, complètement hydrolysé, la proportion massique en polymère d'oxyde de propylène et d'oxyde d'éthylène doit être au minimum de 0,5% et celle en lauryl éther sulfate de sodium au minimum de 1 % ;
- pour 4,5% d'acétate de vinyle polymérisé avec l'alcool vinylique, complètement hydrolysé, la proportion massique en polymère d'oxyde de propylène et d'oxyde d'éthylène doit être au minimum de 0,3%, et celle en lauryl éther sulfate de sodium au minimum de 0,9% ;
- pour 5% d'acétate de vinyle polymérisé avec l'alcool vinylique, complètement hydrolysé, la proportion massique en polymère d'oxyde de propylène et d'oxyde d'éthylène doit être au minimum de 0,2%, et celle en lauryl éther sulfate de sodium au minimum de 0,8%.

Pour ces trois exemples, le pH doit par ailleurs être tamponné entre 3,0 et 4,0.

## Revendications

1. Composition désinfectante et filmogène destinée à être appliquée après une traite sur le trayon d'un bovin, d'un ovin ou d'un caprin comprenant en milieu aqueux :
- 3,5 à 5% en masse de l'acétate de vinyle polymérisé avec l'alcool vinylique ; et
- 0,75 à 2,9 % en masse d'un complexe de polyvinylpyrrolidone et d'iode, **caractérisée en ce qu'**elle comprend en outre :
- 0,2 à 0,6% en masse de polymère d'oxyde de propylène et d'oxyde d'éthylène ; et
- 0,5 à 1% en masse de lauryl éther sulfate de sodium.

2. Composition désinfectante et filmogène selon la revendication 1, **caractérisée en ce que** la proportion massique du polymère d'oxyde de propylène et d'oxyde d'éthylène dans ladite composition est comprise entre 0,25 et 0,35 %.

3. Composition désinfectante et filmogène selon l'une quelconque des revendications 1 et 2, **caractérisée en ce que** ledit acétate de vinyle polymérisé avec l'alcool vinylique est hydrolysé à au moins 98%.

4. Composition désinfectante filmogène selon l'une quelconque des revendications 1 à 3, **caractérisée en ce qu'**elle comprend en outre 0,5 à 2% en masse de PEG-7 glyceryl cocoate.

5. Composition désinfectante et filmogène selon l'une quelconque des revendications 1 à 4, **caractérisée en ce qu'**elle comprend 3 à 10% en masse de glycérine.

6. Composition désinfectante et filmogène selon l'une quelconque des revendications 1 à 5, **caractérisée en ce qu'**elle comprend 0,05 à 1% en masse d'un tampon de pH.

7. Composition désinfectante et filmogène selon l'une quelconque des revendications 1 à 6, **caractérisée en ce qu'**elle comprend 0,1 à 0,5% en masse d'un agent régulateur de pH ou d'un mélange de plusieurs agents régulateurs de pH en mélange, destinés à ajuster le pH de ladite composition entre 4,5 et 5,5.

8. Composition désinfectante et filmogène selon la revendication 7, **caractérisée en ce que** ledit mélange d'agents régulateur de pH comprend de l'hydroxyde de sodium et de l'acide citrique monohydrate.

9. Composition désinfectante et filmogène selon l'une quelconque des revendications 1 à 8, **caractérisée en ce qu'**elle comprend de la gomme de xanthane, destinée à épaissir ladite composition.

10. Composition désinfectante et filmogène selon l'une quelconque des revendications 1 à 9, **caractérisée en ce qu'**elle comprend 0,01 à 0,5% en masse d'allantoïne.

## Patentansprüche

1. Desinfizierende und filmbildende Zusammensetzung, die zum Auftragen nach dem Melken auf die Euterzitze eines Rindes, eines Schafs oder einer Ziege bestimmt ist, umfassend in wässrigem Medium:
- 3,5 bis 5 Ma% mit Vinylalkohol polymerisiertes Vinylacetat; und
- 0,75 bis 2,9 Ma% eines Komplexes aus Polyvinylpyrrolidon und Jod, **dadurch gekennzeichnet, dass** sie ferner umfasst:
- 0,2 bis 0,6 Ma% Propylenoxid- und Ethylenoxidpolymer; und
- 0,5 bis 1 Ma% Natriumlaurylethersulfat.

2. Desinfizierende und filmbildende Zusammensetzung nach Anspruch 1, **dadurch gekennzeichnet, dass** der Massenanteil des Propylenoxid- und Ethylenoxidpolymers in der Zusammensetzung zwischen 0,25 und 0,35 % liegt.

3. Desinfizierende und filmbildende Zusammensetzung nach einem der Ansprüche 1 und 2, **dadurch gekennzeichnet, dass** das mit dem Vinylalkohol polymerisierte Vinylacetat zu mindestens 98 % hydrolysiert ist.

4. Desinfizierende filmbildende Zusammensetzung nach einem der Ansprüche 1 bis 3, **dadurch gekennzeichnet, dass** sie ferner 0,5 bis 2 Ma% PEG-7 Glycerylcocoat umfasst.

5. Desinfizierende und filmbildende Zusammensetzung nach einem der Ansprüche 1 bis 4, **dadurch gekennzeichnet, dass** sie 3 bis 10 Ma% Glycerin umfasst.

6. Desinfizierende und filmbildende Zusammensetzung nach einem der Ansprüche 1 bis 5, **dadurch gekennzeichnet, dass** sie 0,05 bis 1 Ma% eines pH-Puffers umfasst.

7. Desinfizierende und filmbildende Zusammensetzung nach einem der Ansprüche 1 bis 6, **dadurch gekennzeichnet, dass** sie 0,1 bis 0,5 Ma% eines pH-Regulierungsmittels oder eines Gemischs aus mehreren gemischten pH-Regulierungsmitteln umfasst, die zur Einstellung des pH in der Zusammensetzung zwischen 4,5 und 5,5 bestimmt sind.

8. Desinfizierende und filmbildende Zusammensetzung nach Anspruch 7, **dadurch gekennzeichnet, dass** das Gemisch aus pH-Regulierungsmitteln Natriumhydroxyd und Citronensäure-Monohydrat umfasst.

9. Desinfizierende und filmbildende Zusammensetzung nach einem der Ansprüche 1 bis 8, **dadurch gekennzeichnet, dass** sie Xanthan zur Eindickung der Zusammensetzung umfasst.

10. Desinfizierende und filmbildende Zusammensetzung nach einem der Ansprüche 1 bis 9, **dadurch gekennzeichnet, dass** sie 0,01 bis 0,5 Ma% Allantoin umfasst.

## Claims

1. A disinfectant and film-forming composition intended to be applied after milking on the teat of a bovine, ovine or caprine animal comprising in an aqueous medium:
- 3.5 to 5% by weight of vinyl acetate polymerised with vinyl alcohol; and
- 0.75 to 2.9% by weight of a polyvinylpyrrolidone and iodine complex, **characterized in that** it further comprises:
- 0.2 to 0.6% by weight of propylene oxide and ethylene oxide polymer; and
- 0.5 to 1% by mass of sodium lauryl ether sulfate.

2. Disinfectant and film-forming composition according to claim 1, **characterised in that** the mass proportion of the polymer of propylene oxide and ethylene oxide in said composition is between 0.25 and 0.35%.

3. A disinfectant and film-forming composition according to any of claims 1 and 2, **characterised in that** said vinyl acetate polymerised with vinyl alcohol is hydrolysed to at least 98%.

4. A film-forming disinfectant composition according to any of claims 1 to 3, **characterised in that** it further comprises 0.5 to 2% by weight of PEG-7 glyceryl cocoate.

5. Disinfectant and film-forming composition according to any of claims 1 to 4, **characterised in that** it comprises 3 to 10% by weight of glycerin.

6. A disinfectant and film-forming composition according to any of claims 1 to 5, **characterised in that** it comprises 0.05 to 1% by weight of a pH buffer.

7. Disinfectant and film-forming composition according to any of claims 1 to 6, **characterised in that** it comprises 0.1 to 0.5% by weight of a pH regulating agent or a mixture of several pH regulating agents in mixture, intended to adjust the pH of said composition between 4.5 and 5.5.

8. A disinfectant and film-forming composition according to claim 7, **characterised in that** said mixture of pH regulating agents comprises sodium hydroxide and citric acid monohydrate.

9. Disinfectant and film-forming composition according to any of claims 1 to 8, **characterised in that** it comprises xanthan gum, intended to thicken said composition.

10. Disinfectant and film-forming composition according to any of claims 1 to 9, **characterised in that** it comprises 0.01 to 0.5% by weight of allantoin.
